# EUROPEAN PATENT SPECIFICATION

(11) **EP 3 348 240 B1**
(45) Date of publication and mention of the grant of the patent: **17.07.2019**
(21) Application number: 18150762.5
(22) Date of filing: 09.01.2018
(51) Int. Cl.: A61F 5/01

(54) **TUBULAR SLEEVE TO BE USED TOGETHER WITH AN ORTHOSIS**
ROHRFÖRMIGE HÜLSE ZUR VERWENDUNG ZUSAMMEN MIT EINER ORTHESE
MANCHON TUBULAIRE DESTINÉ À ÊTRE UTILISÉ CONJOINTEMENT AVEC UNE ORTHÈSE

(30) Priority: 17.01.2017 SE 1750035
(43) Date of publication of application: 18.07.2018
(73) Proprietor: Camp Scandinavia AB, 254 67 Helsingborg (SE)
(72) Inventor: JONSSON, Maria, 254 30 Helsingborg (SE)
(74) Representative: AWA Sweden AB

(56) References cited:
- WO-A1-01/45598
- US-A- 5 185 000
- US-A- 6 019 741
- US-A- 6 129 695

## Description

### Technical field

The invention relates to a tubular sleeve to be used together with an orthosis.

### Background

Orthoses are often considered to be devices, external of the body, that serve to or are used to alter, modify, or support, structural and/or functional characteristics of the body's skeletal or neuromuscular systems. For example, an orthosis may be used to assist a person, such as a person suffering with foot nerve damage, with walking. Orthoses can provide confinement and support in static situations, such as in retarding further spinal curving of a patient with scoliosis, and in dynamic situations, such as with supporting and influencing gait of a patient suffering from drop foot. An orthosis may also immobilize, limit, steer, guide, or dictate the position or range of movement of a body extremity, a body joint, or a body area. Accordingly, orthoses may be used for e.g. treatment, improved lifestyle and improved comfort.

One example of an orthosis of the above type is an Ankle Foot Orthosis (AFO). An AFO comprises a foot plate, a strut connected to the foot plate and which extends from the foot plate, and a fastening means that is provided for fastening the orthosis to the lower leg of a user. Examples of these types of AFO's are known from e.g. EP1379201B and US 5897515.

An AFO is worn under the clothing, such as trousers or a skirt. In case of trousers, the AFO will typically be concealed under the trousers. In case of a skirt, the AFO may be concealed under leggings, a hose or by a bootleg. It is however not uncommon that users experience a discomfort of having the AFO directly abutting the skin. They can by way of example experience itchiness and chafing. As a measure to avoid or reduce this kind of discomfort it is known to provide a padding that is arranged to cover the skin-abutting portions of the AFO. The padding may be integrated with the AFO or be provided as a detachable padding.

It is also known to use so called undersleeves. Undersleeves are frequently used together with braces of different types in order to protect the skin and also prevent undue sliding of the brace along the limb or joint, such as a knee or elbow. Typically, an undersleeve is provided as a flexible tube-shaped fabric sleeve that has a length corresponding to the longitudinal extension of the limb to be covered. Although using an undersleeve, the brace as such is typically provided with some sort of integrated or detachable padding. Also, in order to conceal the brace, some sort of clothing is required, such as trousers, leggings, stockings or bootlegs. Document US 6,019,741 discloses an elastic stocking which fits over a patient's lower leg and foot and a rigid brace contoured to fit over the front of the patient's shin. The brace is secured to the stocking. Document WO 01/45598 A1 discloses a sleeve comprising a frusto-conical portion.

### Summary of the invention

It is an object of the present invention to solve at least some of the problems mentioned above. More precisely it is an object to provide a simple padding solution to the user of an orthosis, and especially an ankle foot orthosis, AFO, to avoid itchiness and chafing. The padding solution should be easy to fit on the limb, be easy to wash and also allow convenient concealing of the orthosis.

According to a first aspect the invention relates to a tubular sleeve according to claim 1.

Thus, a partly padded tubular sleeve is provided that is fitted to the limb before mounting the orthosis. The tubular sleeve is then folded back over the limb and parts of the orthosis. Thereby one portion of the tubular sleeve, which corresponds to the padded first portion, is arranged between the limb and the orthosis whereas the folded over portion, corresponding to the second portion, is arranged on the exterior side of the orthosis, thereby concealing the same. The parts of the orthosis that are contained in the thus formed interspace between the first and second portions of the tubular sleeve are in fact not only concealed but also held in position against the limb. Accordingly, the tubular sleeve may serve the dual purpose of providing a padding between the limb and the orthosis and firmly positioning the orthosis on the limb. Thereby the AFO must not be of the type being provided with integrated fixing means such as straps but may be an AFO of the type without any integrated fixing means.

By at least the first portion comprising a padding the risk of experiencing itchiness and chafing is reduced and even avoided. Also, the overall comfort may be improved. Further, the ability to provide a simple concealing of major parts of the orthosis, that otherwise would be visible under e.g. a skirt means a lot to the self-confidence of the user. Further, for wearers of trousers, the tendency of the trousers hooking up with the orthosis may be reduced or even eliminated.

The tubular sleeve is easy to exchange and wash, whereby the overall hygiene may be improved.

The padding may have an extension along the longitudinal extension and along a part of the circumference of the first portion of the tubular sleeve corresponding to at least the part of the orthosis that during use of the orthosis is adapted to abut the limb. By restricting the surface extension of the padding to areas where an abutment with the orthosis is intended, the flexibility and ease of fitting the tubular sleeve to a limb is improved.

The padding may have a continuous or dis-continuous surface extension along the longitudinal extension of the first portion of the tubular sleeve and/or along a part of the circumference of the first portion of the tubular sleeve. By restricting the surface extension of the padding to areas where an abutment with the orthosis is intended, the flexibility and ease of fitting the tubular sleeve to a limb is improved.

The tubular sleeve may be made of a woven or knitted elastic material such as lycra or a combination of lycra and polyester.

The padding may be made of woven or knitted textile material, a foamed plastics material, a synthetic rubber material such as chloroprene rubber, a 3D spacer fabric or a fiberfill foam. The density of the padding may be 1-70 shore and more preferred 5-60 shore.

The padding may be perforated. The perforations add to the flexibility of the tubular sleeve and thus to the overall comfort and ease of fitting the same to a limb.

The first and second portions may be joined by sewing, adhesive bonding or welding.

The first and second portions may be made up by at least two panels. The panels may be arranged side by side, in an overlapping relationship or in a partially overlapping relationship.

The orthosis may be an ankle foot orthosis and the limb may be the lower leg.

Accordingly and in summary, a tubular sleeve adapted to be used together with an ankle foot orthosis having a strut adapted to extend along a lower leg of a user is provided. The tubular sleeve having a first frusto-conical portion and a second frusto-conical portion arranged one after the other as seen in a longitudinal extension of the tubular sleeve, and wherein the first frusto-conical portion and the second frusto-conical portions are adapted to be fitted onto the lower leg of the user, at least the first frusto-conical portion comprising a padding having an extension along the longitudinal extension and along a part of the circumference of the first frusto-conical portion of the tubular sleeve, and the second frusto-conical portion being adapted to be folded back over the first portion whereby the strut is received in the thus formed interspace formed between the first and the second frusto-conical portions.

A further scope of applicability of the present invention will become apparent from the detailed description given below. However, the detailed description and examples, while indicating preferred embodiments of the invention, are given by way of illustration only, since various changes and modifications within the scope of the invention will become apparent to those skilled in the art from this detailed description.

Hence, it is to be understood that this invention is not limited to the particular component parts of the device described or steps of the methods described as such device and method may vary. It is also to be understood that the terminology used herein is for purpose of describing embodiments only, and is not intended to be limiting. It must be noted that, as used in the specification and the appended claims, the articles "a," "an," "the," and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings does not exclude other elements or steps.

### Brief description of the drawings

These and other aspects of the present invention will now be described in more detail, with reference to the appended drawings showing embodiments of the invention. The figures are provided to illustrate the general structures of embodiments of the present invention. Like reference numerals refer to like elements throughout.
Fig. 1 discloses schematically a side view of one embodiment of a tubular sleeve according to the invention.
Figs. 2a and 2b disclose the tubular sleeve when used together with an ankle foot orthosis.
Fig. 3 discloses one example of how the tubular sleeve can be made up by a number of panels.
Fig. 4 discloses a second example of how the tubular sleeve can be made up by a number of panels.
Fig. 5 discloses a third example of how the tubular sleeve can be made up by a number of panels.
Fig. 6 discloses a fourth example of how the tubular sleeve can be made up by a number of panels.
Fig. 7 discloses one example wherein the padding has a dis-continuous surface extension.

### Detailed description

Throughout this document reference will be made to geometrical terms such as frusto-conical and trapezoids in order to facilitate the understanding of the underlying idea and realization of the inventive concept. The inventive tubular sleeve is suitable to be tailor-made to fit the body constitution of a specific user. This may result in panels having a design not falling within the strict mathematic definition of the geometrical terms as used in the description. Especially the legs of the trapezoid must not be linear but may be curved.

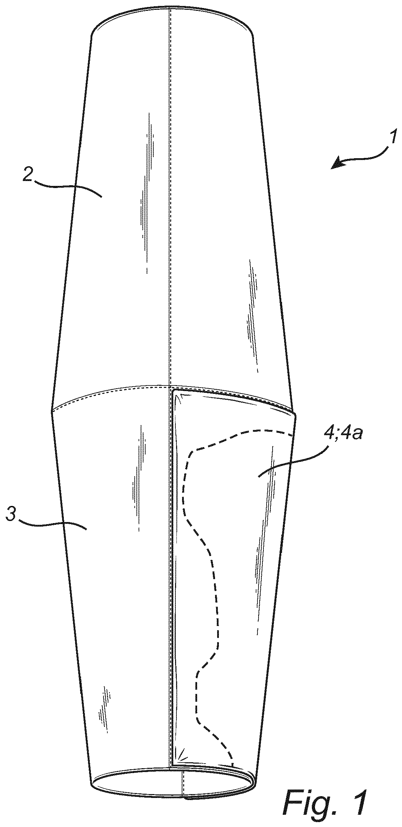

Referring now to Fig.1 the general design of a padding solution in the form of a tubular sleeve 1 is disclosed. The tubular sleeve 1 is adapted to be fitted onto a limb in combination with an orthosis. The tubular sleeve 1 is especially designed to be fit on the lower leg together with an ankle foot orthosis (AFO).

The tubular sleeve 1 comprises a first portion 2 and a second portion 3 which are arranged one after the other as seen in a longitudinal extension of the tubular sleeve 1. The first and second portions 2, 3 are disclosed as having the same longitudinal extension. Still, different longitudinal extensions may be provided.

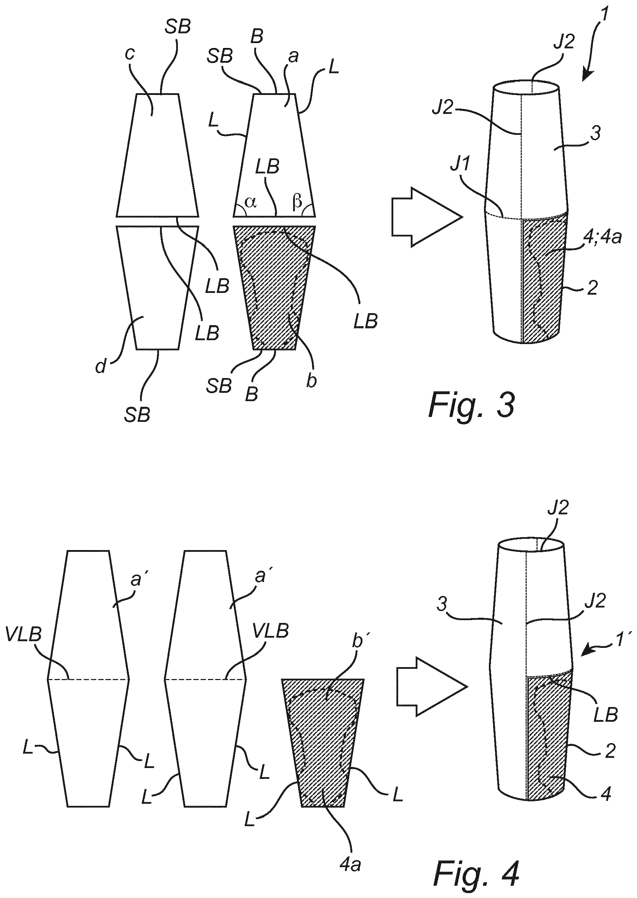

The human lower leg typically has a smaller diameter in the ankle area than in the calf area. In order to account for this constitution, the first and second portions 2, 3 do each have a frusto-conical shape, i.e. one end with a small diameter and one end with a large diameter. The first and second portions 2, 3 merge along the large diameter ends. Depending on how the tubular sleeve 1 is provided, the first and second portions 2, 3 may be seen as a virtual geometrical division or as a physical geometrical division. The latter requires the at least two panels being joined to each other. It is to be understood that the tubular sleeve 1 may be provided in a number of ways. A few, non-limiting examples, will be given below with reference to Figs 3-7.

The first and the second portions 2, 3 are made up by an elastic material. The elastic material may by way of example be a woven or knitted material such as lycra, or a mixture between polyester and lycra. The elastic material may also be made of natural materials such as cotton. The density of the elastic material may differ across the tubular sleeve 1 in order to improve breathing or to locally enhance the concealing properties of the tubular sleeve.

The first portion 2 comprises along at least a part of its circumferential direction a padding 4. The padding 4 may extend along the full or only along a part of the longitudinal extension of the first portion. The padding 4 is provided by a woven or knitted textile material, a foamed plastics material, a synthetic rubber material such as chloroprene rubber, a 3D spacer fabric or a fiberfill foam. A 3D spacer fabric is a three-dimensional knitted spacer fabric. No matter type of material, the material chosen should be flexible. The padding 4 may be provided as a continuous homogenous surface or be provided with perforations or local cut-outs to thereby correspond to the design of the support member and /or any fastening means of the AFO. Also, the padding 4 may be provided with different thickness across its surface area.

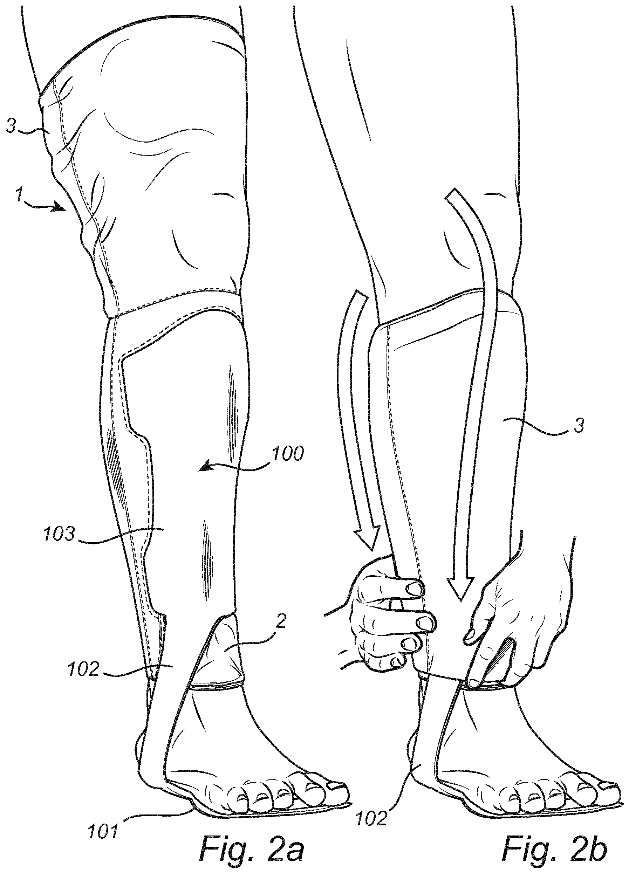

Now turning to Figs. 2a and 2b, the tubular sleeve 1 is illustrated as used on the lower leg together with an AFO 100. A typical AFO 100 comprises a foot plate 101 and a strut 102. The AFO 100 is typically made of a composite material. The strut 102 is connected to the foot plate 101 and is adapted to extend along the lower leg. The strut 102 comprises a support member 103 providing a support along the tibia.

The tubular sleeve 1 is fitted onto the lower leg with the padding 4 abutting the tibia. Then the AFO 100 is arranged on the lower leg with the support member 103 abutting the exterior surface of the padding 4. In this position, the second portion 3 of the tubular sleeve 1 is folded back over the first portion 2. Thereby, the major part of the AFO 100, such as the support member 103, is received and firmly contained and concealed in the interspace formed between the overlapping parts of the first and second portions 2, 3. Thereby the padding 4 will be arranged against the skin whereby any direct contact between the skin and the support member 103 is prevented. Also, as a result of at least the support member 103 of the AFO being firmly contained in the interspace between the overlapping parts of the first and second portions 3, there is no need for the AFO 100 to comprise any fastening means for fastening the orthosis to the lower leg. It is however to be understood that in the event the AFO is of the type comprising fastening means, such as straps, the orthosis is attached to the lower leg by encircling the fastening means around the lower leg before folding back the second portion 3 over the first portion 2. Hence, any such fastening means will be contained and concealed in the interspace.

No matter type of the AFO 100, any slipping between the AFO 100 and the lower leg is prevented or at least reduced. Depending on the longitudinal extension of the first portion 2, contact between the skin and the strut 102 may also be prevented. Accordingly, the risk of chafing and itchiness is reduced.

The degree of concealing depends on the longitudinal extension of the second portion 3. To allow a full concealing, the second portion 3 should have a length as seen in the longitudinal extension corresponding to at least the length of the first portion 2.

The tubular sleeve 1 may be provided in numerous ways with remained function. Starting with Fig. 3 a first embodiment is disclosed. The first portion 2 is made up by two panels b, d and the second portion 3 is made up by two panels a, c. Each panel a-d has the shape of a trapezoid. A trapezoid comprises two parallel edges, known as bases B. The two bases B have different lengths - one long base LB and one short base SB. The two bases B are interconnected by two edges, known as legs L. The legs L may have equal lengths. The angles α, β between a long base LB and a leg L are preferably obtuse such as in the range of 70-85°. As given above the tubular sleeve 1 should be designed to fit the body constitution of the user, whereby the length of the short base SB should correspond to about half the diameter of the ankle and the length of the long base LB should correspond to about half the diameter of the calf. The ratio between the lengths of the short base SB and the long base LB will hence determine the angles α, β.

The two panels b, d and a, c forming parts of the first and second portions 2, 3 respectively are disclosed as being identical. It is to be understood that this must not be the case in order to better fit the body constitution of a user.

The two panels b, d making up the first portion 2 are made of different materials. One panel d is made of an elastic material, such as lycra or a combination of lycra and polyester, whereas the other panel b is made of a padding material 4a such as a woven or knitted textile material, a foamed plastics material, a synthetic rubber material such as chloroprene rubber, a 3D spacer fabric or a fiberfill foam.

The two sections a, c making up the second portion 3 are made of the same material and preferably of the same material as panel d of the first portion 2.

The four panels a-d are joined to each other in order to provide the tubular sleeve 1. A first joint J1 extends in the circumferential direction of the tubular sleeve 1 and interconnects the long bases LB of the respective four panels a-d. Further, two opposing joints J2 extend in the longitudinal direction of the tubular sleeve 1 along the legs L of opposing panels a, c and b, d. The resulting tubular sleeve 1 will accordingly be provided with a first and a second portion 2, 3, each having a frusto-conical shape, with two opposing joints J2 extending in the longitudinal direction of the tubular sleeve 1 and one circumferential joint J1 interconnecting the first and the second portions 2, 3. The tubular sleeve 1 has two opposing open ends. When fitted on a user, the padding 4 is adapted to face and abut the tibia. Further, the two longitudinal joints J2 will extend along the sides of the lower leg. The circumferential joint J1 will form a natural folding line when the second portion 3 is folded back over the first portion 2 to conceal an AFO.

It is to be understood that the joining method depends on the type of material. Possible joining methods may by way of example be sewing, adhesive bonding or welding, depending on the type of material. Combinations of different joining methods may be used.

Now turning to Fig. 4 a second embodiment of the tubular sleeve 1' is disclosed. The tubular sleeve 1' is composed of two panels of a first type a' and one panel of a second type b'.

The first type panel a' has the shape corresponding to a mirrored trapezoid, wherein the mirroring is made along a virtual long base VLB disclosed as a dashed line. The virtual long base VLB has a length corresponding to about half the circumference of the calf. The two first type panels a' are made of an elastic material such as lycra or a combination of lycra and polyester.

The second type panel b' has the shape of a trapezoid. The second type panel b' is made of a padding material 4a such as a woven or knitted textile material, a foamed plastics material, a synthetic rubber material such as chloroprene rubber, a 3D spacer fabric or a fiberfill foam.

The second type panel b' is arranged to overlap one of the first type panels a' with the legs L of the second type b' panel being aligned with the legs L of the first type panel a'. Further, the two first type panels a' are joined to each other along their respective legs L to thereby form two opposing joints J2 extending in the longitudinal direction of the thus formed tubular sleeve 1'.

The long base LB of the second type panel b' will form a natural folding line as the second portion 3 is folded back over the first portion 2 to conceal an AFO. To facilitate folding, the second type panel b' could be joined to one of the first type panels a' along the long base LB of the second type panel b'. To facilitate folding, the second type panel b' may be joined to the first type panel a' along the long base LB of the second type panel b'. When fitted on a lower leg, the padding 4 will abut the tibia and the two longitudinal joints will extend along the sides of the lower leg. The tubular sleeve 1' has two opposing open ends.

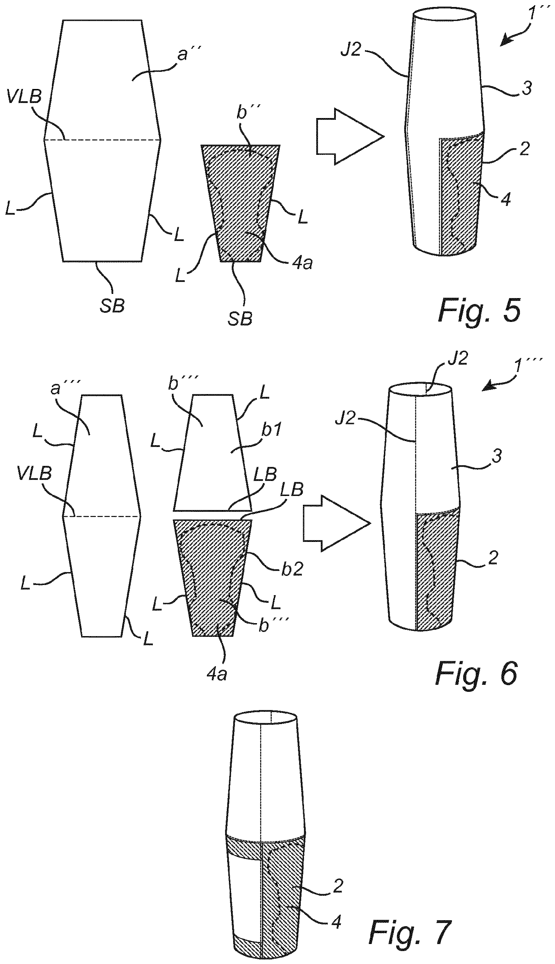

Now turning to Fig. 5 a third embodiment of the tubular sleeve 1" is disclosed. The tubular sleeve 1" is composed of a first type panel a" of an elastic material and a second type panel b" of a padding material 4a. Like previous embodiments, the elastic material may be lycra or a combination of lycra and polyester and the padding material 4a may be a woven or knitted textile material, a foamed plastics material, a synthetic rubber material such as chloroprene rubber, a 3D spacer fabric or a fiberfill foam.

The first type panel a" has the shape corresponding to a mirrored trapezoid, wherein the mirroring is made along a virtual long base VLB. The virtual long base VLB illustrated by a dashed line has a length corresponding to the circumference of the calf. The second type panel b" has the shape of a trapezoid. It is to be understood that the virtual long base VLB may be omitted and that panel a" instead is divided into two panels which are joined to form a joint having an extension corresponding to the virtual base line VLB.

The second type panel b" is arranged on top of the first type panel a" with the short base SB of the second type panel b" being aligned with and centralized along the short base SB of the first type panel a". The second type panel b" is fixedly arranged to the first type panel a" by e.g. sewing along the legs L of the second type panel b". The thus resulting first type panel a" is formed into a tubular sleeve 1" by one single longitudinal joint J2 interconnecting the opposing legs L. The tubular sleeve 1" has two opposing open ends.

When fitted to a lower leg, the long base LB of the second type panel b" will form a natural folding line as the second portion 3 is folded back over the first portion 2 to conceal an AFO. To facilitate folding, the second type panel b" may be joined to the first type panel a" along the long base LB of the second type panel b". The longitudinal joint J2 will in this embodiment be arranged to extend along the calf whereas the padding 4 will extend along the tibia.

Now tuning to Fig. 6, a fourth embodiment of the tubular sleeve 1''' is disclosed. The tubular sleeve 1''' is composed of one panel of a first type a'" and two panels of a second type b'''.

The panel of the first type a'" has the shape corresponding to a mirrored trapezoid, wherein the mirroring is made along the virtual long base VLB which is disclosed by a dashed line. It is to be understood that the virtual long base VLB may be omitted and that panel a'" instead is divided into two panels which are joined to form a joint having an extension corresponding to the virtual long base VLB.

The panel of the first type a'" is made of an elastic material. The virtual long base VLB has a length corresponding to half the circumference of the calf.

The panel of the second type b'" has the shape of a trapezoid with dimensions corresponding the trapezoid on which the first type panel a'" is based. Whereas a first panel b1 of the second type is made of the same material as the panel of the first type, a second panel b2 is made of a padding material 4a.

Like previous embodiments, the elastic material may be lycra or a combination of lycra and polyester and the padding material 4a may be a woven or knitted textile material, a foamed plastics material, a synthetic rubber material such as chloroprene rubber, a 3D spacer fabric or a fiberfill foam.

The two panels b1, b2 of the second type b'" are joined to each other along their respective long bases LB. The thus two joined panels b1, b2 are then joined to the panel of the first type a'" along the legs L to thereby form two opposing joints J2 extending in the longitudinal direction of the thus formed tubular sleeve 1'''. The tubular sleeve 1''' has two opposing open ends.

When fitted on a lower leg, the joint interconnecting the two panels b'" of the second type will form a natural folding line as the second portion 3 is folded back over the first portion 2.

The padding 4 has been illustrated as having a continuous extension. Fig. 7 discloses one example wherein the padding 4 has a dis-continuous surface extension along the longitudinal extension and along a part of the circumference of the first portion 2 of the tubular sleeve. The padding 4 is divided into two areas. One area extends in the circumferential direction and is adapted to cover an intended contact area between any fastening means of the AFO and the first portion 2 of the tubular sleeve. The other area extends in the longitudinal direction and is adapted to cover an intended contact area between the support member of the AFO and the first portion 2 of the tubular sleeve. The two areas of the padding 4 can have different densities.

No matter the different embodiments that have been discussed above, it is to be understood that the diameters of the small and large bases of the panels are preferably chosen to fit the body constitution of a specific user. Thus, the tubular sleeve may be tailor-made.

The legs of the trapezoid shaped panels have been disclosed as being straight. It is to be understood that the legs may be provided with slightly curved extensions depending on the body constitution of the user.

The first and second portions of the tubular sleeve may be provided by different types of elastic materials, with different elastic strengths and with different colors or patterns. The latter applies especially to the second portion which will be visible when the AFO and the tubular sleeve is worn with a skirt.

The first and second portions may as such be seen as a virtual division to better understand the geometry. Thus, even though the tubular sleeve has been exemplified as being constituted by a number of joined panels, it may in principle be provided by circular knitting. The circular knitting may be provided with local areas of increased density or local areas with another material to thereby form a padding.

Not only the first portion, but also the second portion may comprise a padding having an extension along the longitudinal extension and along a part of the circumference of the second portion of the tubular sleeve. The density of the padding of the second portion may be the same or lower than the density of the padding of the first portion.

Therefore, variations to the disclosed embodiments can be understood and effected by the skilled person in practicing the claimed invention, from a study of the drawings, the disclosure, and the appended claims.

## Claims

1. Tubular sleeve (1) adapted to be used together with an orthosis (100) having a strut (102) adapted to extend along a limb, the tubular sleeve (1) having a first portion (2) and a second portion (3) arranged one after the other as seen in a longitudinal extension of the tubular sleeve (1), and wherein
the first portion (2) and the second portions (3) are adapted to be fitted onto the limb,
at least the first portion (2) comprising a padding (4) having an extension along the longitudinal extension and along a part of the circumference of the first portion (2) of the tubular sleeve (1), and
(2), wherein
the first and second portions (2, 3) each have a frusto-conical shape having a long base (LB) and a short base (SB) and wherein the first and second portions (2, 3) merge along their respective long bases (LB) and wherein the second portion (3) is adapted to be folded back over the first portion (2) along said merging respective long bases (LB).

2. The tubular sleeve according to claim 1, wherein the padding (4) has an extension along the longitudinal extension and along a part of the circumference of the first portion (2) of the tubular sleeve (1), which in a condition when the tubular sleeve (1) is used together with an orthosis (100), corresponds to at least the part of the orthosis (100) that is adapted to abut the limb.

3. The tubular sleeve according to claim 1, wherein the padding (4) has a continuous or dis-continuous surface extension along the longitudinal extension of the first portion (2) of the tubular sleeve (1) and along a part of the circumference of the first portion (2) of the tubular sleeve (1).

4. The tubular sleeve according to claim 1, wherein the tubular sleeve (1) is made of a woven or knitted elastic material such as lycra or a combination of lycra and polyester.

5. The tubular sleeve according to claim 1, wherein the padding (4) is made of woven or knitted textile material, a foamed plastics material, a synthetic rubber material such as chloroprene rubber, a 3D spacer fabric or a fiberfill foam.

6. The tubular sleeve according to claim 1, wherein the padding (4) is perforated.

7. The tubular sleeve according to claim 1, wherein the first and second portions (2, 3) are joined by sewing, adhesive bonding or welding.

8. The tubular sleeve according to claim 1, wherein the first and second portions (2, 3) are made up by at least two panels.

9. The tubular sleeve according to claim 1, wherein the orthosis is an ankle foot orthosis and wherein the limb is the lower leg.

10. The tubular sleeve according to any of the previous claims, wherein the tubular sleeve (1) comprises two opposing free ends, said free ends being open.

## Patentansprüche

1. Rohrförmige Hülse (1), die ausgelegt ist, um zusammen mit einer Orthese (100) benutzt zu werden, die eine Verstrebung (102) aufweist, die ausgelegt ist, um sich entlang eines Körperglieds zu erstrecken, wobei die rohrförmige Hülse (1) einen ersten Abschnitt (2) und einen zweiten Abschnitt (3) aufweist, die in einer Längserstreckung der rohrförmigen Hülse (1) betrachtet einer nach dem anderen angeordnet sind, und wobei
der erste Abschnitt (2) und der zweite Abschnitt (3) ausgelegt sind, um an das Körperglied angepasst zu werden,
zumindest der erste Abschnitt (2) eine Polsterung (4) umfasst, die eine Erstreckung entlang der Längserstreckung und entlang eines Teils des Umfangs des ersten Abschnitts (2) der rohrförmigen Hülse (1) aufweist, und, wobei
der erste und der zweite Abschnitt (2, 3) jeweils eine kegelstumpfförmige Form mit einer langen Basis (LB) und einer kurzen Basis (SB) haben und wobei der erste und der zweite Abschnitt (2, 3) sich entlang ihrer jeweiligen langen Basen (LB) verbinden und wobei der zweite Abschnitt (3) ausgelegt ist, um über den ersten Abschnitt (2) entlang der jeweiligen verbundenen langen Basen (LB) zurückgefaltet zu werden.

2. Rohrförmige Hülse nach Anspruch 1, wobei die Polsterung (4) eine Erstreckung entlang der Längserstreckung und entlang eines Teils des Umfangs des ersten Abschnitts (2) der rohrförmigen Hülse (1) hat, welche in einem Zustand, wenn die rohrförmige Hülse (1) zusammen mit einer Orthese (100) verwendet wird, zumindest dem Teil der Orthese (100) entspricht, der ausgelegt ist, um an dem Körperglied anzuliegen.

3. Rohrförmige Hülse nach Anspruch 1, wobei die Polsterung (4) eine durchgehende oder unterbrochene Oberflächenerstreckung entlang der Längserstreckung des ersten Abschnitts (2) der rohrförmigen Hülse (1) und entlang eines Teils des Umfangs des ersten Abschnitts (2) der rohrförmigen Hülse (1) hat.

4. Rohrförmige Hülse nach Anspruch 1, wobei die rohrförmige Hülse (1) aus einem elastischen Gewebe oder einer elastischen Wirkware wie etwa Lycra oder eine Kombination aus Lycra und Polyester besteht.

5. Rohrförmige Hülse nach Anspruch 1, wobei die Polsterung (4) aus einem Textilgewebe oder einer Textilwirkware, einem geschäumten Kunststoffmaterial, einem synthetischen Kautschukmaterial wie etwa Chloroprenkautschuk, einem 3D-Abstandsgewebe oder einem Füllwattenschaum besteht.

6. Rohrförmige Hülse nach Anspruch 1, wobei die Polsterung (4) perforiert ist.

7. Rohrförmige Hülse nach Anspruch 1, wobei der erste und der zweite Abschnitt (2, 3) durch Nähen, Verkleben oder Schweißen zusammengefügt sind.

8. Rohrförmige Hülse nach Anspruch 1, wobei der erste und der zweite Abschnitt (2, 3) aus mindestens zwei Stoffbahnen zusammengesetzt sind.

9. Rohrförmige Hülse nach Anspruch 1, wobei die Orthese eine Knöchel-Fuß-Orthese ist und wobei das Körperglied der Unterschenkel ist.

10. Rohrförmige Hülse nach einem der vorhergehenden Ansprüche, wobei die rohrförmige Hülse (1) zwei gegenüberliegende freie Enden umfasst, wobei die freien Enden offen sind.

## Revendications

1. Manchon tubulaire (1) apte à être utilisé avec une orthèse (100) dotée d'un support (102) apte à s'étendre le long d'un membre, le manchon tubulaire (1) comportant une première section (2) et une seconde section (3) disposées l'une après l'autre vues dans une extension longitudinale du manchon tubulaire (1), et dans lequel
la première section (2) et la seconde section (3) sont aptes à être ajustées sur le membre,
au moins la première section (2) comportant un rembourrage (4) ayant une extension le long de l'extension longitudinale et le long d'une partie de la circonférence de la première section (2) du manchon tubulaire (1), et
les premières et seconde sections (2, 3) ont chacune une forme frustoconique dotée d'une longue base (LB) et d'une courte base (SB) et les premières et seconde sections (2, 3) fusionnent le long de leur longues bases respectives (LB) et la seconde section (3) est apte à être repliée sur la première section (2) le long desdites longues bases respectives fusionnant (LB).

2. Manchon tubulaire selon la revendication 1, dans lequel le rembourrage (4) a une extension le long de l'extension longitudinale et le long d'une partie de la circonférence de la première section (2) du manchon tubulaire (1) qui, dans une situation où le manchon tubulaire (1) est utilisé avec une orthèse (100), correspond au moins à la partie de l'orthèse (100) qui est apte à buter contre le membre.

3. Manchon tubulaire selon la revendication 1, dans lequel le rembourrage (4) a une extension en surface continue ou discontinue le long de l'extension longitudinale de la première section (2) du manchon tubulaire (1) et le long d'une partie de la circonférence de la première section (2) du manchon tubulaire (1).

4. Manchon tubulaire selon la revendication 1, dans lequel le manchon tubulaire (1) est composé d'un matériau élastique tricoté comme du Lycra ou une combinaison de Lycra et de polyester.

5. Manchon tubulaire selon la revendication 1, dans lequel le rembourrage (4) est composé de matériau textile tissé ou tricoté, d'une matière plastique en mousse, d'une matière en caoutchouc synthétique comme du caoutchouc chloroprène, d'un tissu d'espacement 3D ou d'une mousse à base de bourres.

6. Manchon tubulaire selon la revendication 1, dans lequel le rembourrage (4) est perforé.

7. Manchon tubulaire selon la revendication 1, dans lequel les première et seconde sections (2, 3) sont assemblées par couture, liaison adhésive ou soudage.

8. Manchon tubulaire selon la revendication 1, dans lequel les première et seconde sections (2, 3) sont composées d'au moins deux panneaux.

9. Manchon tubulaire selon la revendication 1, dans lequel l'orthèse est une orthèse de cheville et le membre est le bas de la jambe.

10. Manchon tubulaire selon l'une quelconque des revendications précédentes, dans lequel le manchon tubulaire (1) comprend deux extrémités libres opposées, lesdites extrémités libres étant ouvertes.
